Europäisches Patentamt

European Patent Office

Office européen des brevets

(11) Publication number: **0 367 306**
**A2**

## EUROPEAN PATENT APPLICATION

(21) Application number: 89200116.5

(22) Date of filing: 19.01.89

(51) Int. Cl.⁵ **A61K 39/35 , A61K 39/36**

(30) Priority: 04.11.88 ES 8803356

(43) Date of publication of application:
09.05.90 Bulletin 90/19

(84) Designated Contracting States:
AT BE CH DE FR GB GR IT LI LU NL SE

(71) Applicant: CORPORACION BIOLOGICA
FARMACEUTICA, SA (C.B.F. , S.A.)
Poligono Industrial de "Tres Cantos" Calle
del Sol (Parcelas Nos. 8-11)
E-28770 Colmenar Viejo (Madrid)(ES)

(72) Inventor: Berrens, Lubertus
Zeemanlaan 14
NL-3572 ZD Utrecht(NL)

(74) Representative: Smulders, Theodorus A.H.J.,
Ir. et al
Vereenigde Octrooibureaux Nieuwe Parklaan
107
NL-2587 BP 's-Gravenhage(NL)

(54) Procedure for polymerized allergenis production.

㉗ Procedure for the production of antigenie pro-
ducts, polymerized and cross-linked, for medical
treatment of allergies.

The allergenic components of high molecular
weight are polymerized with glutardialdehyde and
the reaction is stoped by addition of glycine or
others aminoacids or primary amines.

The reaction product with molecular weight less
than 100 000 daltons are eliminated by flow filtration,
dialysis, molecular sieving or columnar gel filtration.

The components with molecular weight over 100
000 daltons are concentrated and preserved by
freezing in solution or in the gry state by lyophiliza-
tion.

## PROCEDURE FOR POLYMERIZED ALLERGENIS PRODUCTION

The traditional long-term treatment of pollen hay fever depends on a regimen of injections of aqueous pollen extracts for an extended period of time ("hyposensitization", "desensitization", "immunotherapy"). This form of therapy was introduced by NOON and FREEMAN in 1911, i.e. during a period of growing insight in the immunology of infectious disease and its treatment by vaccination. Accordingly, NOON and FREEMAN must have considered pollen hay fever as a form of infectious disease that might in analogy be treated with vaccines prepared from the causative agents. There is every reason to believe that this notice was incorrect, scientifically, because allergy is not a disease due to infection and, clinically, because the results of this form of treatment have remained dubious over the years. However, injection treatment for hyposensitization with allergenic extracts is still being practiced today. Whatever the outcome of these developments, most allergenic extracts available for immunotherapy at this moment in time still contain the active natural allergens. Consequently, injection treatment with active allergenic extracts in any pharmaceutical formulations requires extreme caution in order to avoid side-effects (e.g. anaphylactic reactions) during prolonged administration. Furthermore, the treatment demands stable and well standardized extracts, which cannot easily be achieved. As alternatives - although likewise along the lines of "vaccinotherapy" preparations have been developed, consisting of active allergens adsorbed to insoluble carriers, e.g. aluminium hydroxide, calcium phosphate, or water-in-oil emulsions. These preparations again rely on the presumption of inducing protective or "blocking" antibodies in the patient. With all these variants, and in the mind of most cliniciens, the aim appears to be the induction of a state of immunological tolerance to allergens in general by generating specific antibodies of other class than IgE. For successful treatment in this sense, the prerequisite therefore must be an intact antigenicity or immunogenicity, preferably associated with a reduced intrinsic allergenicity.

As a major development in the design of safe and stable therapeutic pollen extracts, attempts have been made to reduce the allergeniticy, while retaining full antigenicity of the allergens. An important advance in this field of research has been the preparation and administration of allergenic extracts cross-linked chemically by means of aliphatic dialdehydes, i.e. glutardialdehyde. A group of researchers has demostrated convincingly that glutaraldehyde polymerized pollen extracts provide safe and reliable substitutes in traditional immunotherapy. The therapeutic efficacy, of cours, remains a subject of debate. Polymerized pollen allergens (of grasses, trees or weeds) exibit only 1 % of the active allergenicity of the native allergens (by skin test or RAST-inhibition), although they retain full immunogenicity in man and laboratory animals.

The allergen polymers are soluble in aqueous buffers and may therefore be administered in the same fashion as the traditional aqueous extracts. However, the average molecular size of the polymers is 100 -1000 times larger than the original allergens, so that the products at the same time provide a form of slow-release preparation. Hence, the addition of carriers like aluminium hydroxide is not strictly essential. The polymerized pollen allergens represent safe, stable and reliable products for the treatment of pollen hay fever. The dosage for treatment and the injection schedules may remain essentially the same as with the active pollen extracts. Alternatively, by increasing the initial dosage of the active product, the number of injections before reaching the maintenance dose may be reduced considerably. Clinical improvement has been reported and documented, and the incidence of side-reactions was found to be considerably reduced. Protection from clinical hay fever is reported to have been achieved with a regimen of as little as 9 sequential injections, because the dosage of polymerized pollen could be increased much more rapidly than with the native extracts.

Polymerized pollen extracts may be prepared from any pollen species in the form of soluble and stable preparations. By keeping these products in store in the lyophilized form, any combination of polymerized pollen allergens for individual treatment can easily be provided.

The present Report provides a novel and updated technology for the large-scale production of glutardialdehyde-linked polymers from the pollens allergens of grasses, trees and weeds, as well as the preparation of pharmaceutical products featuring polymerized mite- and animal allergens.

The starting material for bulk polymer production must in all cases consist of the lyophilized high-molecular weight components from homogeneous allergenic source materials extracted with distilled water or with aqueous buffers. These non-dialysable components are further indicated as HMWT-products. The HMWT material is first dissolved in phosphate buffer pH 7,4 to a concentration of 14-16 mg/ml. The buffer may be an ordinary phosphate-saline buffer or be made up according to

0,1 M NaCl

4 mM $KH_2PO_4$

15 mM $Na_2HPO_4.l2 H_2O$

pH = 7,4

The clear solution. clarified if necessary by centrifugation, is stirred on a magnetic stirrer at room temperature, and a freshly prepared solution of 25 % glutaraldehyde is added dropwise in a ratio of 17-19 μl per ml of allergen solution. The mixture is stirred for 6 hours at room temperature, whereupon the reaction is stopped by the addition of 38-40 mg of the amino acid glycine per ml of solution, either as a solid or as the equivalent in ml of a 200 mg/ml solution of glycine in phosphate-saline buffer. Stirring is then continued at low revolution overnight at + 4₀C. For pollen polymers the solution is diluted with distilled water to a minimum of 250 ml and pumped dialysed by tangential flow through an > 100 000 cut-off filter until the dialysate is colourless or optically empty. Finally, the filter is washed clean with a small volume of distilled water, and the collected M> 100 000 polymer fraction is concentrated to volume of about 150-200 ml, frozen by shell-freezing and dried in high vacuum by lyophilization. The freezedried product is stored in the dark at room temperature in an evacuated desiccator over a drying agent, preferably dehydrated silica gel but not phosphorous pentoxide. Under these conditions, it is stable for no longer than one year. Polymer solutions in physiological buffer for human use are made up fresh only when needed.

The following examples may illustrate the above explanation.

Example I - Polymer of the pollen allergens of "Parietaria judaica L."

Extraction of "Parietaria judaica" pollen.

25 grams of pollen preparation was extracted with 200 ml of distilled water with stirring for 2 hours at room temperature. The mixture was centrifuged, and the residue was re-extracted under the same conditions. The combined supernatant extracts were then dialysed exhaustively against distilled water from Visking 10 000 Membranes, and the nondialysable retentate was finally dried by lyophilization to give 870 mg of the HMWT product, i.e. a yield of 3,5 % from dry pollen.

In another batch preparation, 100 grams of Parietaria pollen was extracted as above with 600 ml each, and the final dialysed retentate was lyophilized to yield 2460 mg of the HMWT product, i.e. 2,5 % from dry pollen.

Preparation of the polymer

The lyophilized batch of HMWT material, 435 mg of "Parietaria judaica" pollen, was dissolved in 30 ml of phosphate-buffered saline PBS at pH 7,4, i.e. 14,5 mg/ml. The clear solution was kept agitated on a magnetic stirrer, and 525 μl of fresh 25 % glutardialdehyde solution was added, i.e. 175 μl/ml. Stirring was continued for 7 hours at room temperature. the reaction was then terminated by the addition of 1200 mg of solid glycine; the mixture was left standing overnight at + 4₀C. The solution was then passed through a Pellicon PTHK 100 000 tangential filter fitted into a Millipore system for dialysis and ultrafiltration, and concentrated to 200 ml in the same apparatus. The clear solution containing the components at M> 100 000 daltons was finally dried by lyophilization. The yield of freezedried polymer product was 126 mg.. or 29 % from the HMWT material and was coded.

A second bath was produced by dissolving 2000 mg of "Parietaria judaica" HMWT lyophilized allergenic extract in 140 ml of PBS pH 7,4, i.e. 14,3 mg/ml, and dropwise addition of 2,4 ml of 25% glutardialdehyde solution, i.e. 17,1 μl/ml. The micture was stirred for 6 hours at room temperature, followed by the addition of 5,5 grams of glycine and continued stirring overnight at + 4₀C. After dilution and passage through the Pellicon system, the ultrafiltrate was lyophilized to give 812 mg of polymer product. Yield: 41 % from "Parietaria judaica" HMWT:

Example II: Polymer of birch tree pollen ("Betula alba L")

Extraction of s sample of birch pollen

A sample of 25 grams of dry pollen of birch ("Betula alba L") was defatted with ether and extracted twice with 200 ml each of distilled water as described in Example 1. The combined extracts were dialysed and freezedried to give 568 mg of the HMWT product, i.e. a yield of 2,3 % from dry pollen.

Preparation of the polymer

The birch pollen HMWT extract was dissolved as the total stock of 568 mg in 40 ml PBS at pH 7,4, i.e. 14,2 mg/ml. A volume of 688 μl of a 25% fresh glutardialdehyde solution was then added dropwise with stirring at room temperature, i.e. 17,2 μl/ml. Stirring was then continued for 7 hours at room temperature and the reaction was terminated by the addition of 1.57 grams of glycine. Stirring was continued for 30 minutes at room temperature and overnight at + 4$\varrho$C. The solution was finally dialysed and concentrated on a 100 000 dalton Pellicon tengential filter and dried by lyophilization. The yield was 220 mg of polymer, i.e. 32 % from the active HMWT product.

## Example III: Polymer of "Artemisia vulgaris L." weed pollen.

### Extraction of a sample of "Artemisia vulgaris" pollen

A sample of 25 grams of the dry pollen was defatted with ether and extracted twice with distilled water as described in Example I. The yield of the HMWT product was 2095 mg, i.e. 8,4 % from dry pollen.

### Preparation of the polymer

A quantity of 838 mg of the lyophilized HMWT extract of the pollen of "Artemisia vulgaris" was dissolved in 52 mg of PBS at pH 7,4 i.e. 16 mg/ml. To the clear solution, a total volume of 1000 μl of 25% glutardialdehyde was added dropwise and with stirring, i.e. 19,2 μl/ml. The mixture was stirred for 6 hours at room temperature, whereupon the reaction was stopped by the addition of 2,04 grams of glycine. After a further 30 minutes at room temperature, the micture was stirred overnight at + 4$\varrho$C, and then dialysed and concentrated through a Pellicon N> 100 000 daltons filter pad. After lyophilization, the yield was 255 mg of the polymer product, i.e. 30 % from HMWT.

## Example IV: Polymer of the pollen allergens of "Phleum pratense L."

### Extraction of Phleum pratense pollen

A quantity of 100 grams of the pure and dried pollen was defatted with ethylether in a Soxhlet apparatus until the extract was colourless (about 6 hours). The pollen sample was then air-dried, weighed and extracted with stirring for 2 hours at room temperature with 500 ml of distilled water. The mixture was centrifuged, and the residue was re-extracted in the same fashion. The combined supernatant extracts were dialysed exhaustively against distilled water at + 4$\varrho$C (Visking membrane, exclusion M = 10 000) and the non-dialysable retentate was finally dried by lyophilization to give 7300 mg of th HMWT product, i.e. a yield of 7,3 %

### Preparation of the polymer

An aliquot of 3650 mg of HMWT was dissolved in 240 ml of PBS buffer pH 7,4, i.e. at 15,2 mg/ml. To the clear solution, a total volume of 4,3 ml of 25% glutardialdehyde was added dropwise and with stirring, i.e. 18,0 μl/ml. Stirring was continued for 6 Hours at room temperature, and 9,4 grams of solid glycine was then added, i.e. 39mg.ml. After a further 30 minutes at room temperature, the mixture was stored overnight at 4$\varrho$C, and then dialysed and concentrated through a Pellicon M> 100 000 tangential filter pad. After lyophilization of the > 100 000 dalton retentate, the yield of the polymer product was 2410 mg, i.e. 66 % from the parent HMWT product.

## Example V : Polymer of mite-body allergens of "Dermatophagoides pteronyssinus"

### Extraction of mites "Dermatophagoides pteronyssinus"

8,617 grams of crude mite source material was extracted with 100 ml PBS pH 7,4 + $10^{-4}$ NaN$_3$ for two days at + 4$\varrho$C. The mixture was centrifuged in the cold and the insoluble residue was re-extracted with 100 ml of the same fluid for 4 Hours. The combined supernatants were dialysed from Visking membranes (exclusion 10 000) against 3 changes of distilled water. The non-dialysable retentate was finally lyophilized after shell-freezing to give 575 mg of the HMWT preparation, i.e. a yield of 6,7 % from the mite source material.

### Preparation of the polymer

The lyophilized HMWT material was dissolved in a quantity of 500 mg in 35 ml PBS pH 7,4, i.e. at 14,3 mg/ml and to the clear solution a volume of 605 ul 25 % glutardialdehyde was added dropwise with agitation on a magnetic stirrer, i.e. 17,3 ul/ml. Stirring was continued for 7 hours at room temperature, whereupon the reaction was terminated by addition of 1,4 grams of glycine. The mixture was left overnight at + 4$\varrho$C, then dialysed against several changes of distilled water from Visking dialysis tubing (exclusion: 10 000 daltons) and finally dried by lyophilization. Yield: 337 mg crude polymer, i.e. 67,4 % from HMWT.

The crude polymer was further fractionated by applying 300 mg dissolved in 2,5 ml of PBS pH 7,4 to a d x h = 25 x 90 cm chromatographic column packed with Sephacryl S-300 Superfine suspended in the same buffer. The column had in a previous run bean calibrated with the standard proteins, viz. catalase (M = 232 000), aldolase (M = 158 000)- ,bovine serum albumin (M = 67 000) and ovalbumin (M = 43 000) The effluent from the column was run through a flow-through quartz cell for the continuous measurement of the UV - light transmission at 280 nm and collected by means of an automatic fraction collector. Fractions were pooled to prepare two pools, viz.: Pool I comprising the material with M 100 000 daltons and Pool II comprising the polymerized material of M 100 000 daltons. These pools were separately dialysed against distilled water from Visking tubing and subsequently lyophilized to give 62 mg of Pool I Polymer (M 100 000), yield 20,7 % and 186 mg of Pool II (M 100 000), yield 62 %.

## Example VI: Polymer of mite allergens from "Dermatophagoides farinae"

### Extraction mites "Dermatophagoides farinae"

8,5 grams of mite source material was extracted with 100 ml PBS for 2 days with stirring as described for D. pteronyssinus. The mixture was centrifuged, the insoluble residue was re-extracted with 100 ml PBS, for 3 hours at + 4ₒC and centrifuged again. The supernatants of both extractions were combined.

A sample of 100 ml of the unprocessed combined supernatants was stored frozen at -20ₒC as a document. The rest of the supernatant was dialysed against several changes of distilled water from Visking membrane. The nondialysable HMWT-fraction was finally lyophilized to give 600 mg, yield = 8,1%

### Preparation of the polymer

A quantity of 500 mg of the lyophilized HMWT material from whole body source material of "Dermatophagoides farinae" was dissolved in 32 ml phosphate-buffer saline pH 7,4, i.e. at 15,6 mg/ml. To the clear solution, agitated on a magnetic stirrer, 603 µl of a 25 % glutardialdehyde solution was added dropwise, i.e. 18,8 µl/ml. Stirring was continued for 6 hours at room temperature, followed by the addition of 1,23 grams of glycine and continued stirring for 1 hour. The mixture was left overnight at + 4ₒC and then dialysed from Visking dialysis tubing (exclusion 10 000) against distilled water and lyophilized. The yield of the crude polymer product was 420 mg, or 84 % from HMWT. A 70 mg sample was retained.

Of the polymer product, a quantity of 350 mg was dissolved in 2,5 ml PBS pH 7,4 and this solution was applied to a calibrated d x h = 25 x 90 cm column packed with Sephacryl S-300 Superfine in the same buffer (see Example V). Elution with PBS resolved two penks of UV-absorbing material at 200 nm, which were separated by means of an automatic fraction collector and combined into an M 100 000 Pool I and an M 100 000 Pool II. The Pool I solution was dialysed from Visking membranes against distilled water and subsequently lyophilized to give 85 mg of the desired therapeutic polymer, i.e. a recovery of 24,3 % from the crude polymer, 20.4 from the direct HMWT extract, or 1,7 % from the original mite source material.

## Example VII: Polymer of the dander allergens of the cat "Felix domesticus".

### Preparation of the active allergenic extract

10 grams of acetone-treated mixed cat dender source material was extracted 3 times with about 70 ml each of PBS, pH 7,4. The centrifuged and combined extracts were dyalized from Visking tubing (exclusion 10000) for 24 hours against three changes of distilled water and the non dialysable retentate was finally lyophilized to give 546 mg of the HMWT preparation, i.e. a yield of 55 % from crude cat dander.

### Preparation of the polymer

A quantity of 100 mg of the lyophilized non-dialysable material (M> 10000) HMWT produced from mixed dander material of domestic cats was dissolved in 7,0 ml of phosphate-buffered saline PBS at pH 7,4 i.e. 14,3 mg/ml. The solution was agitated slowly on a magnetic stirrer and 121 µl of 25 % glutardialdehyde solution was carefully added dropwise, i.e. 17,3 µl/ml. The mixture was stirred for 6 hours at room temperature, followed by addition of 276 mg of glycine and further agitation for 1 hour at room temperature and overnight at + 4ₒC. The deep yellow-brown coloured solution was dried directly by lyophilization, yielding 310 mg of a salt-containing crude polymer product. This preparation was taken up in 4 ml of PBS pH 7,4 and applied to a d x h = 25 x 40 cm chromatographic column packed with Sephacryl S-300 Superfine in PBS.

The column was eluted with the same buffer; effluent fractions were scanned for UV-absorption at 280 nm. The high molecular size fractions displaying residual enzyme activity were pooled and dried by lyophilization "without prior dialysis" to yield 35 mg of the desired therapeutic polymer, i.e. 35 % from HMWT, or 1,9 % from the original cat dander source material.

## Claims

1. - Procedure for the large-scale production of polymerized and cross-linked antigenic products for the medical treatment of allergies.

2. - Procedure for the production according to Claim 1 of inmunogenic products from allergenic source material consisting of the lyophilized high-molecular weight components of active allergenic extracts.

3.-Procedure for the production according to Claims 1 and 2 wherein solutions of the high-molecular weight allergenic components are mixed and polymerized with glutardialdehyde in defined conditions.

4. - Procedure for the production according to Claims 1 and 3 wherein the polymerizations process with glutardialdehyde is terminated by the addition of glycine, other monoamino acids, or primary amines.

5. - Procedure for the production according to Claims 1 to 4 wherein crosslinked and polymerized reaction products having a molecular weight less than 100 000 Daltons are eliminated by means of tangential flow filtration, dialysis, molecular sieving, or columnar gel filtration.

6. - Procedure for the production according to Claims 1 to 5 wherein the polymerized and cross-slinked products having a molecular size of over 100 000 Daltons are separated and concentrated by means of tangential flow dialysis and/or molecular sieving, and are preserved by freezing in solution or in the dry state by lyophilization.

7. - Procedure for the production according to Claims 1 to 6 of polymerized, crosslinked and molecularly defined antigenic and inmunogenic products for the medical treatment of allergies.